# EUROPÄISCHE PATENTSCHRIFT

(11) **EP 0 726 890 B1**
(45) Veröffentlichungstag und Bekanntmachung des Hinweises auf die Patenterteilung: **12.05.1999**
(21) Anmeldenummer: 94930979.3
(22) Anmeldetag: 26.10.1994
(51) Int. Cl.: C07C 231/12, C07C 233/36, C07C 233/38, C07C 233/05, C11D 1/90, C07D 233/54

(54) **VERFAHREN ZUR HERSTELLUNG VON DETERGENSGEMISCHEN**
PROCESS FOR PRODUCING DETERGENT MIXTURES
PROCEDE DE PREPARATION DE MELANGES DETERGENTS

(30) Priorität: 02.11.1993 DE 4337324
(43) Veröffentlichungstag der Anmeldung: 21.08.1996
(73) Patentinhaber: Henkel Kommanditgesellschaft auf Aktien, 40191 Düsseldorf (DE)
(72) Erfinder: BIGORRA LLOSAS, Joaquim, E-08025 Barcelona (ES); PI, Rafael, E-08400 Granollers (ES); PRAT QUERALT, Ester, E-08328 Alella (ES)
(86) Internationale Anmeldenummer: EP9403520
(87) Internationale Veröffentlichungsnummer: WO9512571

(56) Entgegenhaltungen:
- EP-A- 0 020 907
- EP-A- 0 243 619
- EP-A- 0 302 329
- EP-A- 0 353 580
- EP-A- 0 492 228

## Beschreibung

### Gebiet der Erfindung

Die Erfindung betrifft ein Verfahren zur Herstellung von Detergensgemischen, bei dem man Amine in Gegenwart von ausgewählten nichtionischen Tensiden betainisiert.

### Stand der Technik

Amphotere bzw. zwitterionische Tenside sind ausgesprochen hautverträglich und weisen ausgezeichnete Reinigungseigenschaften auf. Sie eignen sich daher in besonderer Weise zur Konfektionierung einer Vielzahl von oberflächenaktiven Produkten. Zu ihrer Herstellung geht man im einfachsten Fall von sekundären oder tertiären Aminen aus, die mit Natriumchloracetat zu Alkylbetainen umgesetzt werden. Die Umsetzung von Fettsäureamidoaminen oder Imidazolinen mit Natriumchloracetat führt zur Bildung von amphoteren Tensiden vom Typ der Glycinate, wird als Alkylierungsmittel Acrylsäureester eingesetzt, bilden sich Aminopropionate. Verbindungen der genannten Art sind in einer Vielzahl von Übersichtsartikeln beschrieben, von denen an dieser Stelle nur **Parf.Cosm.Arom. 70, 67 (1986), HAPPI, 70, (Nov.1986)** und **Soap Cosm.Chem.Spec. 46, (Apr.1990)** genannt sein sollen.

Ein besonderes Anliegen bei der Herstellung der amphoteren bzw. zwitterionischen Tenside besteht darin, möglichst hochkonzentrierte, jedoch fließfähige Produkte herzustellen, um auf diesem Wege Transport- und Lagerkosten zu minimieren. Des weiteren ist es erforderlich, die an sich ausgezeichnete Hautverträglichkeit der Produkte nicht durch Spuren an nicht umgesetzten Alkylierungsmittel zu beeinträchtigen und eine Phasentrennung infolge eines zu hohen Elektrolytgehaltes zu vermeiden.

In der Vergangenheit hat es nicht an Vorschlägen gemangelt, eines oder mehrere der genannten Probleme zu lösen. So ist aus der **DE-A1 2926479** (Th.Goldschmidt) ein Verfahren zur Herstellung von Alkylamidobetainen bekannt, bei dem man den Restgehalt an Alkylierungsmittel minimiert, indem man die Reaktion bei einem pH-Wert von 7,5 bis 10,5 durchführt. In die gleiche Richtung weist die Lehre der **DE-A1 2063424** (Rewo), die die pH-Regulierung für die Alkylierung von Imidazolinen beschreibt.

Gemäß der **DE-A1 4040887** (Th.Goldschmidt) wird zur Herstellung von fließfähigen wäßrigen Betaindispersionen vorgeschlagen, die Quaternierung in wäßriger oder alkoholischer Lösung unter Zusatz anionischer Tenside durchzuführen. Aus der **GB-A 2022125** ist femer bekannt, daß sich C_{12/14}-Kokosalkyldimethylamin in Gegenwart einer wäßrigen Lösung von Natriumlaurylsulfat mit Natriumchloracetat alkylieren läßt. In beiden Schriften wird jedoch ausdrücklich darauf hingewiesen, daß die Anwesenheit von Wasser während der Alkylierung unbedingt erforderlich ist, da andemfalls nicht mehr fließfähige Produkte mit einer lamellaren Flüssigkristallstruktur entstehen würden. Nach den genannten Verfahren können somit zwar hochkonzentrierte, jedoch keine wasserfreien Tensidpasten erhalten werden.

Ein weiteres Verfahren zur Herstellung von fließ- und pumpfähigen Betainen mit einem Aktivsubstanzgehalt von mindestens 70 Gew.-% ist aus der **EP-B1 0243619** (Th.Goldschmidt) bekannt. Hierin wird vorgeschlagen, als Ausgangsstoffe ausschließlich solche Amidoamine einzusetzen, die einen Schmelzpunkt von maximal 30°C aufweisen und die Quatemierung mit Kalium- bzw. Ammoniumchloracetat in einem organischen Lösungsmittel, das höchstens 20 Gew.-% Wasser enthalten darf, durchzuführen. Für eine technische Realisierung kommt ein solches Verfahren indes wohl kaum in Betracht, da die erforderliche Abtrennung des Lösungsmittels vom Wertprodukt nach Abschluß der Alkylierung mit einem erheblichen technischen Aufwand verbunden wäre. Gegenstand der **EP-A1 0353580** ist ein Verfahren zur Alkylierung von sekundären oder tertiären Aminen in Anwesenheit von nichtionischen Tensiden, bei dem die Reaktion jedoch in wäßriger oder wäßrig-alkoholischer Lösung durchgeführt wird.

Aus der **EP-B1 0302329** (Th.Goldschmidt) ist femer bekannt, daß sich fließfähige Betainpasten mit einem Gehalt von ca. 40 Gew.-% herstellen lassen, indem man die nach konventionellen Quatemierungsverfahren erhaltenen wäßrigen Betainlösungen auf den gewünschten Wassergehalt eindampft und den pH-Wert anschließend durch Zugabe von Mineralsäure auf 1 bis 4,5 einstellt. In der **EP-A2 0353580** (Th.Goldschmidt) wird vorgeschlagen, konzentrierte, fließfähige wäßrige, gegebenenfalls niedere aliphatische Alkohole enthaltende Lösungen von Betainen herzustellen, in dem man die Quatemierung in wäßriger oder wäßrig-alkoholischer Lösung unter Zusatz einer solchen Menge nichtionischer Tenside durchführt, daß die resultierende Lösung einen Niotensidgehalt von vorzugsweise 3 bis 20 Gew.-% aufweisen. Als geeignete nichtionische Tenside kommen hierbei vor allem Fettsäurepolyethylenoxidester in Betracht. Als weitere geeignete - jedoch ausdrücklich nicht bevorzugte - Tenside werden Fettalkoholalkoxylate mit einem HLB-Wert im Bereich von 14 bis 20 genannt. Die Lehre dieser Druckschrift geht jedoch dahin, die Quatemierung in Gegenwart von Wasser und/oder Alkoholen - vorzugsweise Ethanol - durchzuführen und zur Viskositätsemiedrigung den Betainen vor der Quatemierung, vorzugsweise jedoch im Anschluß daran, äußerst hydrophile nichtionische Tenside, beispielsweise Polysorbate mit 120 Ethylenoxid-Einheiten, zuzusetzen. Auch nach diesem Verfahren lassen sich somit keine wasserfreien Produkte herstellen. Ein weiterer Nachteil besteht darin, daß zwangsläufig Compounds von Betainen und äußerst hydrophilen nichtionischen Tensiden erhalten werden, wobei letztere aufgrund ihrer mangelnden Schaumentwicklung für kosmetische Produkte oder Geschirrspülmittel wenig geeignet sind.

Die Aufgabe der Erfindung hat somit darin bestanden, amphotere Tenside in wasserfreier Form zur Verfügung zu stellen und dabei die Nachteile des Stands der Technik zu vermeiden.

### Beschreibung der Erfindung

Gegenstand der Erfindung ist ein Verfahren zur Herstellung von Detergensgemischen, bei dem man sekundäre oder tertiäre Amine in Gegenwart von ethoxylierten Fettsäurepartialglyceriden, jedoch ohne Zusatz von Wasser bzw. organischen Lösungsmitteln in an sich bekannter Weise mit Halogencarbonsäuren bzw. deren Alkalisalzen oder Estem umsetzt.

Überraschenderweise wurde gefunden, daß sich ethoxylierte Fettsäurepartialglyceride in besonderer Weise als Lösungsmittel für die Betainisierung von sekundären bzw. tertiären Aminen eignen, so daß der Einsatz von Wasser, Alkoholen oder anderen organischen Lösungsmitteln an dieser Stelle vermieden wird.

### Amine

Als Aminkomponenten kommen sekundäre, insbesondere tertiäre Amine in Betracht. Geeignete Ausgangsstoffe sind beispielsweise **Di-, vorzugsweise Trialkylamine** der Formel **(I)**, 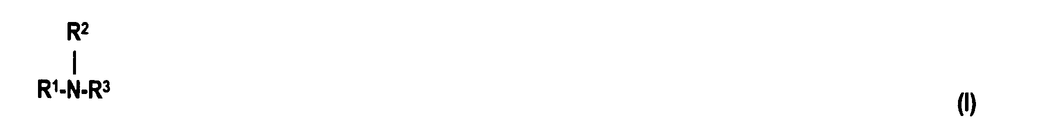 in der R¹ für Alkyl- undloder Alkenylreste mit 6 bis 22 Kohlenstoffatomen, R² für Wasserstoff oder Alkylreste mit 1 bis 4 Kohlenstoffatomen und R³ für Alkylreste mit 1 bis 4 Kohlenstoffatomen steht. Typische Beispiele sind Hexylmethylamin, Hexyldimethylamin, Octyldimethylamin, Decyldimethylamin, Dodecylmethylamin, Dodecyldimethylamin, Dodecylethylmethylamin, C_{12/14}-Kokosalkyldimethylamin, Myristyldimethylamin, Cetyldimethylamin, Stearyldimethylamin, Stearylethylmethylamin, Oleyldimethylamin, C_{16/18}-Talgalkyldimethylamin sowie deren technische Gemische. Als weitere Einsatzstoffe kommen auch **Amidoamine** der Formel **(II)** in Betracht, 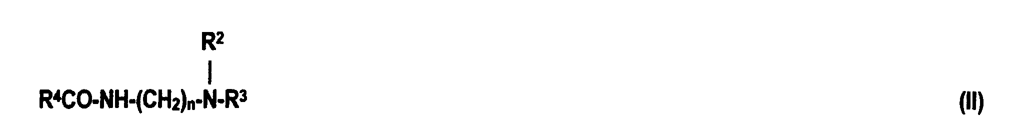 in der R⁴CO für einen aliphatischen Acylrest mit 6 bis 22 Kohlenstoffatomen und 0 oder 1 bis 3 Doppelbindungen, n für 0 oder Zahlen von 1 bis 3 steht und R² und R³ die oben angegebenen Bedeutungen haben. Amidoamine stellen bekannte Stoffe dar, die nach den einschlägigen Verfahren der präparativen Chemie erhalten werden können. Ein Verfahren zu ihrer Herstellung besteht beispielsweise darin, Fettsäuren mit Diaminen zu amidieren. Typische Beispiele sind Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethylaminopropylamin. Bevorzugt ist der Einsatz von C_{12/14}-Kokosfettsäure-N,N-dimethylaminoprcpylamid. Weiterhin kommen als geeignete Amine **Imidazoline** der Formel **(III)** in Betracht, 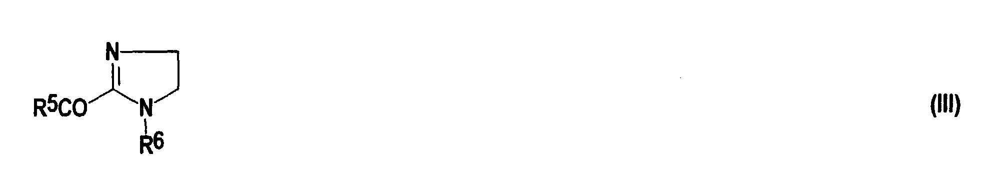 in der R⁵CO für einen aliphatischen Acylrest mit 6 bis 22 Kohlenstoffatomen und 0 oder 1 bis 3 Doppelbindungen und R⁶ für R⁵CO oder einen N(CH₂)₂OH-Rest steht. Auch bei diesen Substanzen handelt es sich um bekannte Stoffe, die beispielsweise durch cyclisierende Kondensation von 2 Mol Fettsäure mit Ethylendiamin erhalten werden können. Typische Beispiele sind Kondensationsprodukte der oben genannten Fettsäuren mit Ethylendiamin oder Aminoethylethanolamin, vorzugsweise Imidazoline auf Basis von Laurinsäure oder wiederum C_{12/14}-Kokosfettsäure.

### Ethoxylierte Fettsäurepartialglyceride

Bei den im Sinne des erfindungsgemäßen Verfahrens einzusetzenden ethoxylierten Fettsäurepartialglyceriden handelt es sich vorzugsweise um Anlagerungsprodukte von durchschnittlich 1 bis 10, insbesondere 5 bis 9 Mol Ethylenoxid an technische Gemische von Fettsäuremono- und -diglyceriden mit 8 bis 22 Kohlenstoffatomen in der Fettsäurekette. Typische Beispiele sind Anlagerungsprodukte von durchschnittlich 5 bis 9 Mol Ethylenoxid an Kokosfettsäuremono- und -diglyceride, insbesondere Anlagerungsprodukte von durchschnittlich 7 Mol Ethylenoxid an technisches C_{8/18}-Kokosfettsäuremonoglycerid (Cetiol® HE, Verkaufsprodukt Henkel KGaA, Düsseldorf/FRG). Die Amine und die ethoxylierten Fettsäurepartialglyceride können im molaren Verhältnis von 15 : 1 bis 1 : 1 eingesetzt werden. Im Hinblick auf die anwendungstechnischen Eigenschaften der resultierenden Detergensgemische hat sich jedoch ein molares Einsatzverhältnis von 10 : 1 bis 2 : 1 als optimal erwiesen.

### Betainisierung

Als Mittel zur Betainisierung der Amine können Halogencarbonsäuren bzw. deren Alkalisalze oder Ester eingesetzt werden. Aus Gründen der leichten Verfügbarkeit ist der Einsatz von Natriumchloracetat bevorzugt. Die Amine und die Halogencarbonsäuren bzw. deren Salze können im molaren Verhältnis von 1 : 0,8 bis 1:1,3, vorzugsweise 1:1 bis 1:1,2 eingesetzt werden. Die Betainisierung der Amine kann in an sich bekannter Weise durchgeführt werden. Im Gegensatz zu den Verfahren des Stands der Technik ist die Anwesenheit eines weiteren Lösungsmittels, insbesondere Wasser oder Alkohol, ausdrücklich nicht erforderlich. Üblicherweise wird die Alkylierung bei Temperaturen im Bereich von 70 bis 105°C durchgeführt und ist innerhalb von 1 bis 10, vorzugsweise 3 bis 7 h beendet. Im Hinblick auf eine möglichst vollständige Abreaktion des Alkylierungsmittels hat es sich als vorteilhaft erwiesen, die Reaktion bei einem pH-Wert von 7 bis 11, vorzugsweise 8 bis 10 zu führen. Eine weitere Möglichkeit zur Minimierung des Restgehaltes an Alkylierungsmittel besteht femer darin, der Reaktionsmischung nach Beendigung der Alkylierung einen definierten Überschuß an Aminosäuren, insbesondere Glycin zuzusetzen, wie dies in der **DE-A1 3939264** (Henkel) beschrieben wird. Die Betainisierung wird in Abwesenheit von Wasser durchgeführt. Falls die nach dem erfindungsgemäßen Verfahren erhältlichen Detergensgemische jedoch nicht in wasserfreier Form, sondem als Konzentrate in den Handel gelangen sollen, können die Produkte nach der Betainisierung zu niedrigviskosen Konzentraten mit einem Feststoffgehalt von beispielsweise 30 bis 60 Gew.-% verdünnt werden.

### Detergensgemische

Nach dem erfindungsgemäßen Verfahren lassen sich Detergensgemische erhalten, die mit Wasser zu hochkonzentrierten, niedrigviskosen Produkten mit einem Feststoffgehalt von 30 bis 60 Gew.-% verdünnt werden können und stabil sind, d. h., auch bei längerer Lagerung ist weder eine Entmischung noch eine Salzabscheidung zu beobachten. Ein weiterer Vorteil ist darin zu sehen, daß insbesondere die wasserfreien Gemische gegenüber mikrobiellen Befall ausreichend stabilisiert sind, so daß der Zusatz typischer Konservierungsmittel nicht erforderlich ist. Dies macht die Compounds insbesondere für kosmetische Produkte interessant. Die Detergensgemische besitzen eine ausgezeichnete Reinigungsleistung sowie ein gutes Schaum- und Emulgiervermögen. Sie können als Compounds in den Handel gebracht und vor Ort zu einer Rezeptur verarbeitet oder auch direkt zusammen mit anderen Tensiden und Inhaltsstoffen konfektioniert werden. Wäßrige Compounds der Zusammensetzung
30 bis 40 Gew.-% Betain,
10 bis 50 Gew.-% ethoxyliertes Fettsäuremonoglycerid
0 bis 10 Gew.-% freie Fettsäure,
0 bis 10 Gew.-% Salz und
40 bis 45 Gew.-% Wasser
- mit der Maßgabe, daß sich alle Prozentangaben zu 100 Gew.-% ergänzen - erweisen sich als niedrigviskos, besitzen ein ausgezeichnetes Reinigungs-, Schaum- und Emulgiervermögen, sind dermatologisch gut verträglich, biologisch leicht abbaubar und lassen sich bei Zusatz weiterer typischer Inhaltsstoffe beispielsweise zu einem hervorragenden Handgeschirrspülmittel konfektionieren.

### Gewerbliche Anwendbarkeit

Die nach dem erfindungsgemäßen Verfahren erhältlichen Detergensgemische weisen ausgezeichnete Detergenseigenschaften und eine hohe dermatologische Verträglichkeit auf; zudem sind sie vollständig biologisch abbaubar. Sie lassen sich mit Wasser zu lagerstabilen und niedrigviskosen Konzentraten mit einem Aktivsubstanzgehalt von 55 bis 60 Gew.-% verdünnen. Die nach dem erfindungsgemäßen Verfahren erhältlichen Detergensgemische können zur Herstellung oberflächenaktiver Mittel dienen, als da sind: Wasch-, Spül- und Reinigungsmittel sowie Produkte zur Haar- und Körperpflege. Die Detergensgemische können als solche oder aber nach Verdünnung mit Wasser eingesetzt werden und dann in Mengen von 1 bis 90, vorzugsweise 10 bis 75 Gew.-% - bezogen auf die Mittel - enthalten sein.

### Beispiel

In einem 1-m³-Rührkessel mit Dampfheizung wurden 310 kg (665 mol) eines Anlagerungsproduktes von durchschnittlich 7 Mol Ethylenoxid an ein technisches C_{8/18}-Kokosfettsäuremonoglycerid (Cetiol® HE, Henkel KGaA, Düsseldorf/FRG) vorgelegt, auf 85°C erhitzt und unter Rühren mit 158 kg (1350 mol) Natriumchloracetat versetzt. Innerhalb von 1 h wurden 409 kg (1196 mol) gehärtetes C_{12/14}-Kokosfettsäureamidopropyl.N,N-dimethylamin zugesetzt und die Mischung bei 90°C gehalten, wobei der pH-Wert auf 8,5 bis 9 eingestellt wurde. Nach Beendigung der Zugabe ließ man 5 h weiterreagieren, bis die Aminzahl den Wert 7 erreicht hatte und nicht weiter abnahm. Es wurde ein Produkt folgender Zusammensetzung erhalten:
55 Gew.-% Betain,
38 Gew.-% Kokosfettsäuremonoglycerid+7EO,
5 Gew.-% Kokosfettsäure und
2 Gew.-% Salz.

## Patentansprüche

1. Verfahren zur Herstellung von Detergensgemischen, bei dem man sekundäre oder tertiäre Amine in Gegenwart von ethoxylierten Fettsäurepartialglyceriden, jedoch ohne Zusatz von Wasser bzw. organischen Lösungsmitteln in an sich bekannter Weise mit Halogencarbonsäuren bzw. deren Alkalisalzen oder Estem umsetzt.

2. Verfahren nach Anspruch 1, **dadurch gekennzeichnet,** daß man sekundäre oder tertiäre Amine der Formel (I) einsetzt, 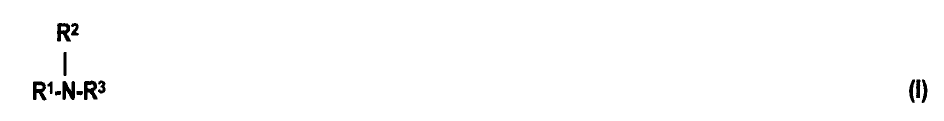 in der R¹ für Alkyl- und/oder Alkenylreste mit 6 bis 22 Kohlenstoffatomen, R² für Wasserstoff oder Alkylreste mit 1 bis 4 Kohlenstoffatomen und R³ für Alkylreste mit 1 bis 4 Kohlenstoffatomen steht.

3. Verfahren nach Anspruch 1, **dadurch gekennzeichnet**, daß man Amidoamine der Formel (II) einsetzt, 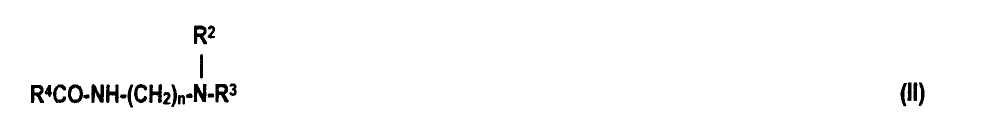 in der R⁴CO für einen aliphatischen Acylrest mit 6 bis 22 Kohlenstoffatomen und 0 oder 1 bis 3 Doppelbindungen, n für 0 oder Zahlen von 1 bis 3 steht und R² und R³ die oben angegebenen Bedeutungen haben.

4. Verfahren nach Anspruch 1, **dadurch gekennzeichnet,** daß man Imidazoline der Formel **(III)** einsetzt, 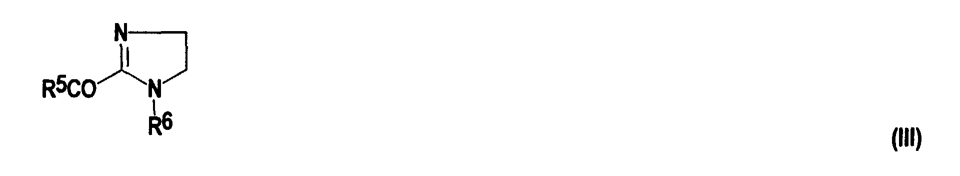 in der R⁵CO für einen aliphatischen Acylrest mit 6 bis 22 Kohlenstoffatomen und 0 oder 1 bis 3 Doppelbindungen und R⁶ für R⁵CO oder einen N(CH₂)₂OH-Rest steht.

5. Verfahren nach den Ansprüchen 1 bis 4, **dadurch gekennzeichnet,** daß man Anlagerungsprodukte von durchschnittlich 1 bis 10 Mol Ethylenoxid an technische Gemische von Fettsäuremono- und Diglyceriden mit 8 bis 22 Kohlenstoffatomen in der Fettsäurekette einsetzt.

6. Verfahren nach den Ansprüchen 1 bis 5, **dadurch gekennzeichnet,** daß man die Amine und die ethoxylierten Fettsäurepartialglyceride im molaren Verhältnis von 15:1 bis 1:1 einsetzt.

7. Verfahren nach den Ansprüchen 1 bis 6, **dadurch gekennzeichnet** daß man Natriumchloracetat einsetzt.

## Claims

1. A process for the production of detergent mixtures in which secondary or tertiary amines are reacted in known manner with halocarboxylic acids or alkali metal salts or esters thereof in the presence of ethoxylated fatty acid partial glycerides, but in the absence of water and organic solvents.

2. A process as claimed in claim 1, characterized in that secondary or tertiary amines corresponding to formula **(I)**: 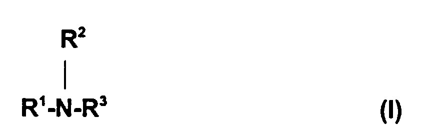 in which R¹ represents alkyl and/or alkenyl radicals containing 6 to 22 carbon atoms, R² represents hydrogen or alkyl radicals containing 1 to 4 carbon atoms and R³ represents alkyl radicals containing 1 to 4 carbon atoms, are used.

3. A process as claimed in claim 1, characterized in that amidoamines corresponding to formula **(II)**: 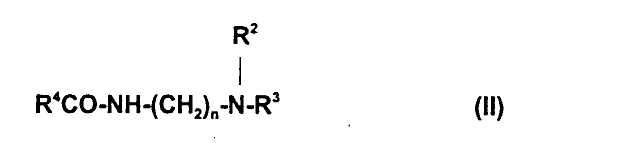 in which R⁴CO is an aliphatic acyl radical containing 6 to 22 carbon atoms and 0 or 1 to 3 double bonds, n is 0 or a number of 1 to 3 and R² and R³ are as defined above,
are used.

4. A process as claimed in claim 1, characterized in that imidazolines corresponding to formula **(III)**: 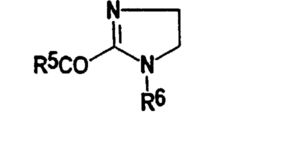 in which R⁵CO is an aliphatic acyl radical containing 6 to 22 carbon atoms and 0 or 1 to 3 double bonds and R⁶ has the same meaning as R⁵ CO or is an N(CH₂)₂OH group.

5. A process as claimed in claims 1 to 4, characterized in that addition products of, on average, 1 to 10 moles of ethylene oxide with technical mixtures of fatty acid monoglycerides and diglycerides containing 8 to 22 carbon atoms in the fatty acid chain are used.

6. A process as claimed in claims 1 to 5, characterized in that the amines and the ethoxylated fatty acid partial glycerides are used in a molar ratio of 15:1 to 1:1.

7. A process as claimed in claims 1 to 6, characterized in that sodium chloroacetate is used.

## Revendications

1. Procédé de fabrication de mélanges détergents dans lequel on fait réagir des amines secondaires ou tertiaires en présence de glycérides partiels d'acide gras éthoxylés, cependant sans addition d'eau ou de solvants organiques, d'une manière connue en soi avec des acides halogénocarboxyliques ou leurs sels de métal alcalin ou avec leurs esters.

2. Procédé selon la revendication 1,
caractérisé en ce qu'
on met en oeuvre des amines secondaires ou tertiaires de formule I : 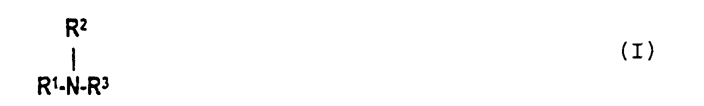 dans laquelle R¹ représente un radical alkyle ou alkényle ayant de 6 à 22 atomes de carbone R² représente de l'hydrogène ou un radical alkyle ayant de 1 à 4 atomes de carbone et R³ représente un radical alkyle ayant de 1 à 4 atomes de carbone.

3. Procédé selon la revendication 1,
caractérisé en ce qu'
on met en oeuvre des amidoamines de formule (II) : 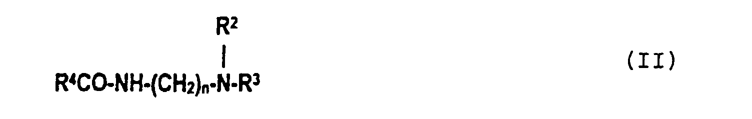 dans laquelle R⁴CO représente un reste acyle aliphatique ayant de 6 à 22 atomes de carbone et 0 ou de 1 à 3 double liaisons, n représente 0 ou des nombres allant de 1 à 3 et R² et R³ ont les significations fournies ci-dessus.

4. Procédé selon la revendication 1,
caractérisé en ce qu'
on met en oeuvre des imidazolines de formule (III) : 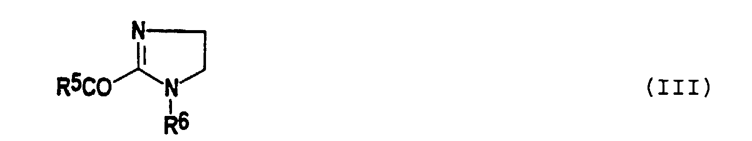 dans laquelle R⁵CO représente un reste acyle aliphatique ayant de 6 à 22 atomes de carbone et 0 ou de 1 à 3 double liaisons et R⁶ représente R⁵CO ou un reste N(CH₂)₂OH.

5. Procédé selon les revendications 1 à 4,
caractérisé en ce qu'
on met en oeuvre des produits d'addition de, en moyenne 1 à 10 mol d'oxyde d'éthylène sur des mélanges industriels de mono- et de diglycérides d'acide gras ayant de 8 à 22 atomes de carbone dans la chaîne d'acides gras.

6. Procédé selon les revendications 1 à 5,
caractérisé en ce qu'
on met en oeuvre les amines et les glycérides partiels d'acide gras éthoxylés dans un rapport molaire allant de 15:1 à 1:1.

7. Procédé selon les revendications 1 à 6,
caractérisé en ce qu'
on met en oeuvre du chloracétate de sodium.
